# EUROPEAN PATENT APPLICATION

(11) **EP 4 368 239 A1**
(43) Date of publication of application: **15.05.2024**
(21) Application number: 22206318.2
(22) Date of filing: 09.11.2022
(51) Int. Cl.: A61N 1/05, A61N 1/368, A61N 1/365

(54) **IMPLANTABLE MEDICAL STIMULATION DEVICE AND METHOD FOR OPERATING AN IMPLANTABLE MEDICAL STIMULATION DEVICE FOR PERFORMING A CARDIAC PACING**

(71) Applicant: BIOTRONIK SE & Co. KG, 12359 Berlin (DE)
(72) Inventor: Dörr, Thomas, 12437 Berlin (DE); Kolberg, Gernot, 12161 Berlin (DE)
(74) Representative: Biotronik Corporate Services SE

(57) **Abstract**

An implantable medical stimulation device (1) for performing a cardiac pacing comprises a generator device (12), an electrode lead (10, 11) connected to the generator device (12) and extending from the generator device (12), and an electrode pole arrangement comprising at least a first electrode pole (102) and a second electrode pole (103), wherein the first electrode pole (102) is configured to penetrate cardiac tissue during implantation and the second electrode pole (103) is arranged on the lead body (100) at a location proximal to the first electrode pole (102). The processing circuitry (120) is configured to generate a stimulation signal and to selectively provide said stimulation signal to one out of at least two combinations of electrode poles of the electrode pole arrangement, wherein in a first combination of the at least two combinations of electrode poles the first electrode pole (102) is used as cathode and in a second combination of the at least two combinations of electrode poles the second electrode pole (103) is used as cathode.

## Description

The present invention relates to an implantable medical stimulation device for performing a cardiac pacing according to the preamble of claim 1 and to a method for operating an implantable medical stimulation device for performing a cardiac pacing.

An implantable medical stimulation device of this kind comprises a generator device comprising processing circuitry for processing electrical signals, an electrode lead connected to the generator device and extending from the generator device, and an electrode pole arrangement comprising at least a first electrode pole and a second electrode pole. The electrode lead comprises a lead body forming a distal end to be arranged, during implantation of the electrode lead, on cardiac tissue within a patient's heart. The first electrode pole of the electrode pole arrangement is configured to penetrate cardiac tissue during implantation. The second electrode pole is arranged on the lead body at a location proximal to the first electrode pole.

With common electrode arrangements of leads of implantable medical stimulation devices, an injection of stimulation signals generally is possible at the surface of intra-cardiac tissue, an electrode being in contact with intra-cardiac tissue in order to allow an injection of stimulation energy into the tissue. With new approaches for example for providing a stimulation in case of a so-called left bundle block, it may be desired to provide for an excitation in a localized fashion in the region of the so-called left bundle branch, also denoted as left bundle branch area pacing (LBBAP), which requires to engage with intra-cardiac tissue in the range of the septum of the heart and to place an electrode in the vicinity of the left bundle branch, such that stimulation energy may be specifically injected into the conductive structure of the left bundle branch. As this requires a piercing of the septum, there is a general desire to provide for an anchoring of an implantable medical device on intra-cardiac tissue which is easy to establish and allows for a spatially differentiated excitation of tissue, in particular in the context of a left bundle branch area pacing.

In particular, when introducing an electrode, for example arranged on a lead, from the right ventricle into the septum in order to reach towards the left bundle branch, the electrode must be inserted into the tissue to reach a substantial depth in order to come to lie in the vicinity of the left bundle branch. This comes with the inherent risk that a piercing structure on which the electrode is arranged may pierce through the septum and may reach into the left ventricle. In addition, when placing an electrode on the septum to reach to the left bundle branch, there is a risk of dislocation, for example when the electrode is arranged on an anchoring device in the shape of a screw, such that an electrical coupling in between the electrode and the left bundle branch may deteriorate over time due to for example a displacement of the screw, potentially leading to a capture loss.

WO 2008/058265 A2 discloses a cardiac stimulation system and method which allow to deliver a left ventricle stimulator from a right ventricle lead system in the right ventricle chamber, into a right side of the septum at a first location, and transmuscularly from the first location to a second location along the left side of the septum. The left ventricle stimulator is fixed at the second location for transmuscular stimulation of the left ventricular conduction system. A biventricular simulation system further includes a right ventricle stimulator also delivered by the right ventricle lead system to the first location along the right side of the septum for right ventricular stimulation.

US 2009/0276000 A1 discloses a method for delivering physiological pacing by selecting an electrode implant site for sensing cardiac signals, which is in proximity to the hearts intrinsic conduction system. An arrangement of multiple electrodes herein is arranged on a tip of a lead.

It is an object of the instant invention to provide an implantable medical stimulation device and a method for operating an implantable medical stimulation device which allow for an easy implantation and operation, in particular in order to provide for a left bundle branch pacing operation.

This object is achieved by means of an implantable medical stimulation device comprising the features of claim 1.

Preferably, according to an embodiment of the present invention, the first electrode pole comprises a helical shape arranged on the distal end of the lead body. The helical shaped first electrode pole is configured to be screwed into cardiac tissue during implantation. Alternatively, the first electrode pole comprises a needle shape arranged on the distal end of the lead body. Both shapes are designed to facilitate penetration or entering of the cardiac tissue, followed by controlled and precise forwarding of the electrode lead in the cardiac tissue. In an embodiment of the present invention, the electrode lead comprises a helical shape at the location of the second electrode pole in case the first electrode pole has a needle shape. The helical shape may provide stability and fixation for the needle shaped first electrode pole.

Accordingly, the processing circuitry is configured to generate a stimulation signal and to selectively provide said stimulation signal to one out of at least two combinations of electrode poles of the electrode pole arrangement, wherein in a first combination of the at least two combinations of electrode poles the first electrode pole is used as cathode and in a second combination of the at least two combinations of electrode poles the second electrode pole is used as cathode.

The generator device, the electrode lead and the electrode pole arrangement placed thereon together form an implantable medical stimulation device which, in an implanted state, is operative to carry out a pacing action, in particular a left bundle branch area pacing (LBBAP).

In the context of providing for a left bundle branch area pacing (LBBAP), an electrode lead is implanted for example in the right ventricle in the region of the septum such that the distal end of the lead body of the electrode lead is placed on the septum in between the right ventricle and the left ventricle. On the distal end of the lead body, herein, an electrode pole of an electrode pole arrangement is placed, which comprises a helical shape and serves to couple with tissue at the septum in order to provide for an electrical excitation during a pacing action.

For providing for a left bundle branch area pacing, the helical electrode pole of the electrode pole arrangement is to be placed within tissue such that it couples to the conductive structure within the septum of the heart, specifically the left bundle branch extending from the so-called atrioventricular node along the septum and within myocardial tissue around the vertex of the left ventricle. Accordingly, during implantation the helical electrode pole arranged on the distal end of the lead body is screwed into tissue, such that the electrode pole engages with tissue and electrically couples with tissue and, in addition, provides for a mechanical anchoring of the distal end on tissue.

Because the helical first electrode pole is easily engagement with tissue, an anchoring of the distal end of the electrode lead on tissue may easily be established, which at the same time cause a coupling of the electrode pole to tissue. During implantation, herein, the first electrode pole may be brought into a depth within the septum in between the right ventricle and the left ventricle such that the first electrode pole is placed in the vicinity of the left bundle branch in order to couple to the conductive structure of the left bundle branch. In addition, also the second electrode pole, for example formed by a ring electrode circumferentially extending about the lead body at a location proximal to the first electrode pole, may come into contact with tissue such that also the second electrode pole is electrically coupled to tissue, for example in the right portion of the septum in the vicinity of the right ventricle.

In order to provide for a stimulation, the processing circuitry is configured to generate a stimulation signal and to selectively provide the stimulation signal to one out of a multiplicity of combinations of electrode poles. Hence, different combinations of electrode poles of the electrode pole arrangement may be used to provide for a cardiac stimulation. By being able to selectively switch between different combinations of electrode poles, a spatially differentiated excitation may be established, which may be selectively adapted to stimulate different structures, such as the right ventricle or the left ventricle.

Within the different combinations, different electrode poles of the electrode pole arrangement are used as cathode. For injecting stimulation signals into tissue, typically a pair of electrode poles is used, one of the electrode poles serving as cathode and the other electrode pole serving as anode. By means of the cathode electrons are injected into tissue, and correspondingly electrons are collected at the anode. Stimulation hence is strongest in the vicinity of the cathode.

In particular, the processing circuitry may switch between a first combination of electrode poles and a second combination of electrode poles to output stimulation signals. In the first combination of electrode poles, the first electrode pole is used as cathode and another electrode pole, for example the second electrode pole, is used as anode. In contrast, in the second combination of electrode poles the second electrode pole is used as cathode and another electrode pole, for example the first electrode pole, is used as anode.

By selectively switching between the different combinations of electrode poles, hence, different excitation vectors for injecting stimulation signals into tissue are employed, such that, by means of the different excitation vectors, different tissue regions may be selectively excited.

This for example allows to selectively excite different conductive structures within tissue. If the first electrode pole is implanted to couple to the left bundle branch, and the second electrode pole is implanted to rest in the vicinity of the right bundle branch, by means of using different combinations of electrode poles a selective excitation of the left bundle branch and right bundle branch may be achieved, such that a biventricular stimulation of the left ventricle and the right ventricle by using a single, bipolar electrode lead may become possible.

In one embodiment, the first combination may use the second electrode pole as anode. Within the first combination, hence, an excitation vector is spanned between the first electrode pole serving as cathode and the second electrode pole serving as anode.

In one embodiment, the second combination may use the first electrode pole as anode. Within the second combination, hence, an excitation vector is spanned between the second electrode pole serving as cathode and the first electrode pole serving as anode. The second combination thus spans an excitation vector opposite to the excitation vector of the first combination.

In one embodiment, the electrode pole arrangement comprises a third electrode pole in addition to the first electrode pole and the second electrode pole. Whereas the first electrode pole is formed by a helical screw arranged at the distal end of the lead body of the electrode lead and the second electrode pole for example is formed by a ring electrode arranged on the lead body proximally with respect to the first electrode pole, the third electrode pole may be formed by the housing of the generator device.

In one embodiment, in at least one out of the at least two combinations of electrode poles of the electrode pole arrangement the third electrode pole may serve as anode. For example, in a third combination of electrode poles the first electrode pole may be used as cathode in which case the third electrode pole may serve as an anode. Alternatively or in addition, in a fourth combination of electrode poles the second electrode pole may be used as cathode and the third electrode pole may serve as an anode. For the third combination of electrode poles and for the fourth combination of electrode poles, hence, the first electrode pole and the second electrode pole on the electrode lead in a unipolar fashion are used as a stimulation electrode, the housing of the generator device serving as an anode and hence as a counter electrode for the unipolar stimulation electrode on the electrode lead.

In one embodiment, the first electrode pole is configured to rest, in an implanted state of the implantable medical stimulation device, within cardiac tissue of the septum of the patient's heart in the vicinity of the left bundle branch. The second electrode pole, which is arranged proximally on the lead body with respect to the first electrode pole and for example is shaped as a ring electrode circumferentially extending about the lead body, in turn is configured to rest within cardiac tissue of the septum in the vicinity of the right bundle branch. Hence, the first electrode pole and the second electrode pole, in an implanted state of the implantable medical stimulation device, shall be placed in the region of different conductive structures within the septum of the patient's heart, namely the left bundle branch and the right bundle branch, such that by means of the different electrode poles an excitation of different conductive structures within the septum may be achieved.

By selectively injecting stimulation signals using the first electrode pole as cathode or by using the second electrode pole as cathode, hence, different conductive structures may be excited, such that by a single, bipolar electrode lead a biventricular excitation of the left ventricle via the left bundle branch and the right ventricle via the right bundle branch may be established.

In one embodiment, the generator device comprises a switching device to selectively switch between the different combinations of electrode poles of the electrode pole arrangement. The switching device may for example be an electronic switching device, for example comprising an arrangement of transistors, such that, controlled by the processing circuitry, it may be selectively switched between different combinations of electrode poles in order to provide for a dedicated, spatially differentiated excitation.

In one embodiment, the processing device is configured to sequentially, within a cardiac cycle, generate a first stimulation signal to be output using the first combination and a second stimulation signal to be output using the second combination. The processing device herein may, beneficially, be configured to generate the first stimulation signal and the second stimulation signal at a defined time distance. By controlling the time distance, hence, a dedicated excitation of the left ventricle and the right ventricle may be achieved, wherein the time distance may be set according to a desired delay between the right ventricular excitation and the left ventricular excitation.

It is to be noted that within a cardiac cycle the first stimulation signal may be output prior to the second stimulation signal, or the second stimulation signal may be output prior to the first stimulation signal. The order of stimulation signals to be output by the different combinations of electrode poles within a cardiac cycle may be freely adapted according to a desired stimulation scheme.

In one embodiment, the processing device is configured to switch from one out of the at least one two combinations of electrode poles of the electrode pole arrangement to another out of the at least two combinations of electrode poles of the electrode pole arrangement based on a processing of at least one sense signal sensed using the electrode pole arrangement.

By means of sensed cardiac signals, for example a capture loss may be identified, based on which the processing device may be configured to switch from a currently used combination of electrode poles to another combination of electrode poles.

In one embodiment, based on sensed cardiac signals a QRS waveform in a recorded electrocardiogram signal may be analyzed, and the processing circuitry may be configured to switch from a currently used combination of electrode poles to another combination of electrode poles if a change in the QRS waveform is identified, for example a broadening in the QRS complex. For example, the processing circuitry may be configured to switch between different combinations of electrode poles and select that combination of electrode poles which yields the most narrow QRS complex.

In one embodiment, the first electrode pole and the second electrode pole are arranged with respect to each other at a distance, measured along a longitudinal axis along which the lead body generally extends, less than 10 mm, preferably in a range between 7 to 8 mm. The first electrode pole is formed by a helical structure on the distal end of the lead body and longitudinally protrudes from the distal end, such that by means of the helical structure the first electrode pole may be screwed into tissue in order to anchor the electrode lead on tissue and advance the electrode lead into tissue. The second electrode pole for example may be formed by a ring electrode circumferentially extending about the lead body, the second electrode pole being arranged proximally with respect to the first electrode pole on the lead body. By spatially separating the first electrode pole and the second electrode pole along the longitudinal axis and by enabling the processing circuitry to switch between different combinations of electrode poles for injecting stimulation signals into tissue, a spatially separated, dedicated excitation of differentiated spatial structures may be achieved.

The first electrode pole and the second electrode pole may, when measured along the longitudinal axis, have approximately the same length, for example a length in between 2 mm to 10 mm, for example between 3 mm to 5 mm.

In one embodiment, the generator device comprises a first bipolar connector having two first electric contact elements and the electrode lead comprises a second bipolar connector comprising two second electric contact elements. The first connector and the second connector may for example each be a so-called IS-1 connector. The first connector and the second connector are connected, in an operative state of the implantable medical stimulation device, with each other for establishing an operative connection in between the processing circuitry, the first electrode pole and the second electrode pole. By enabling the processing circuitry to switch between different combinations of electrode poles, a switchable, selective stimulation may be established, for example allowing for a biventricular excitation using a single bipolar electrode lead.

The implantable medical stimulation device may for example be a one-chamber or two-chamber implantable pulse generator (IPG), or a one-chamber or two-chamber implantable cardioverter defibrillator (ICD).

In another aspect, a method for operating an implantable medical stimulation device for performing a cardiac pacing comprises: providing a generator device comprising processing circuitry for processing electrical signals; providing an electrode lead connected to the generator device and extending from the generator device, the electrode lead comprising a lead body forming a distal end to be arranged, during implantation of the electrode lead, on cardiac tissue within a patient's heart; providing an electrode pole arrangement comprising at least a first electrode pole and a second electrode pole, wherein the first electrode pole is configured to be penetrate cardiac tissue during implantation, wherein the second electrode pole is arranged on the lead body at a location proximal to the first electrode pole; and generating, using the processing circuitry, a stimulation signal and selectively providing said stimulation signal to one out of at least two combinations of electrode poles of the electrode pole arrangement, wherein in a first combination of the at least two combinations of electrode poles the first electrode pole is used as cathode and in a second combination of the at least two combinations of electrode poles the second electrode pole is used as cathode.

The advantages and advantageous embodiments described above for the implantable medical stimulation device equally also apply to the method, such that it shall be referred to the above in this respect.

The idea of the invention shall subsequently be described in more detail with reference to the embodiments shown in the figures. Herein:
- Fig. 1: shows a schematic view of an implantable medical stimulation device having a generator device and electrode leads;
- Fig. 2: shows a schematic drawing of a distal end of an electrode lead in an implanted state;
- Fig. 3: shows a schematic view of an electrode pole arrangement spanning different excitation vectors;
- Fig. 4: shows a schematic view of connectors of a generator device and an electrode lead to be connected to the generator device; and
- Fig. 5: shows a schematic view of a distal end of an electrode lead.
- Fig. 6: shows a schematic cross-sectional view of two embodiments of the electrode lead according to the invention.

Subsequently, embodiments of the invention shall be described in detail with reference to the drawings. In the drawings, like reference numerals designate like structural elements.

It is to be noted that the embodiments are not limiting for the invention, but merely represent illustrative examples.

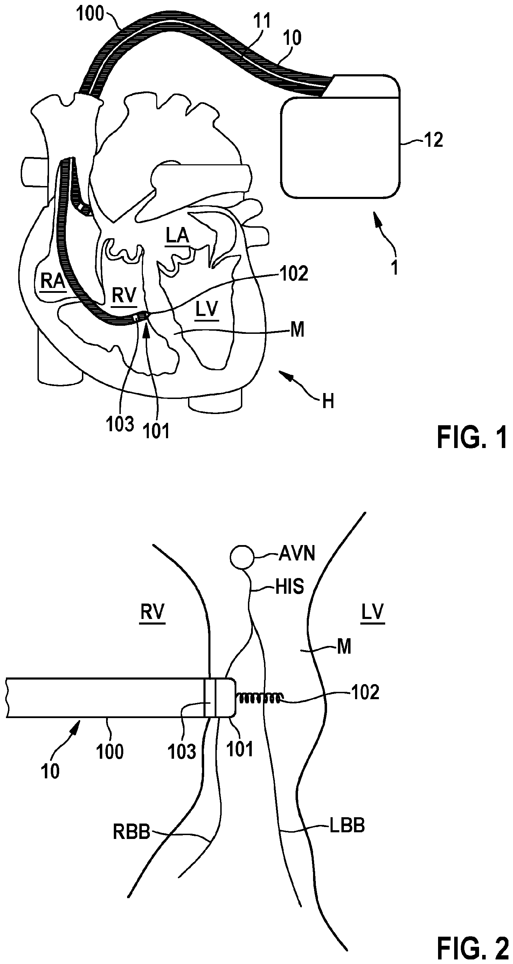

Fig. 1 shows, in a schematic drawing, the human heart H comprising the right atrium RA, the right ventricle RV, the left atrium LA and the left ventricle LV. An implantable medical stimulation device 1 is implanted in a patient, the implantable medical stimulation device 1 comprising a generator 12 connected to leads 10, 11 extending from the generator 12 through the superior vena V into the patient's heart H. By means of the leads 10, 11, electrical signals for providing a pacing action in the heart H shall be injected into intra-cardiac tissue potentially at different locations within the heart, and sense signals may be received.

In the embodiment of Fig. 1, an electrode lead 10 is implanted into the heart H such that it extends into the right ventricle RV of the heart H and, at a distal end 101 of a lead body 100, is arranged on intra-cardiac tissue at the septum M in between the right ventricle RV and the left ventricle LV of the heart H. An electrode lead 11 in turn is implanted such that it reaches into the right atrium RA.

An implantable medical stimulation device 1 as concerned herein may generally be a cardiac stimulation device such as a cardiac pacemaker device such as one-chamber or two-chamber implantable pulse generator (IPG), or a one-chamber or two-chamber implantable cardioverter defibrillator (ICD). A stimulation device of this kind may comprise a generator 12, as shown in Fig. 1, which may be subcutaneously implanted in a patient at a location remote from the heart H, and one or multiple leads 10, 11 extending from the generator 12 into the heart H for emitting stimulation signals in the heart H or for obtaining sense signals at one or multiple locations from the heart H. The leads 10, 11 each form a generally longitudinal, tubular body 100, which reaches into the heart H and is anchored at a location of interest within the heart H.

Referring now to Fig. 2, the implantable medical stimulation device 1 as described herein in particular shall serve to provide a so-called left bundle branch area pacing, in short LBBAP. For this, the electrode lead 10 is implanted such that the lead body 100, with the distal end 101, is placed on tissue on the septum M such that it engages with tissue and reaches into tissue in order to couple to the left bundle branch LBB which, as part of the conductive structure of the patient's heart H, is coupled via the so-called His bundle HIS to the atrioventricular node AVN and runs in parallel to the right bundle branch RBB. The left bundle branch LBB extends within myocardial tissue around the vertex of the left ventricle LV and conducts excitation signals for exciting tissue in the region of the left ventricle LV, whereas the right bundle branch RBB extends within myocardial tissue around the vertex of the right ventricle RV and conducts excitation signals for exciting tissue in the region of the right ventricle RV.

In the shown embodiment, the electrode lead 10 comprises a first electrode pole 102 which is arranged on and protrudes from the distal end 101 of the lead body 100. The electrode pole 102 is formed by a helical screw and is shaped such that it may be screwed into tissue in order to electrically couple to tissue and provide for a mechanical anchoring of the electrode lead 10 on tissue.

In addition, the electrode lead 10 comprises a second electrode pole 103 which is formed by a ring electrode arranged proximally with respect to the first electrode pole 102 on the lead body 100 of the electrode lead 10.

In an implanted state, as shown in Fig. 2, the electrode pole 102 formed by the helical screw is engaged with tissue and reaches into tissue such that it electrically couples to the conductive structure within the myocardial tissue of the septum M, in particular the left bundle branch LBB, in order to enable an excitation of the conductive structure by injecting stimulation signals into the conductive structure. At the same time, the second electrode pole 103 may electrically contact tissue in that it fully or at least partially rests within tissue and hence electrically couples to tissue, in particular the conductive structure of the right bundle branch RBB.

During implantation, the electrode lead 10 is inserted, from the region of the right ventricle RV, into tissue such that the electrode pole 102 at the distal end 101 reaches a sufficient insertion depth within the tissue in order to couple to a desired conductive structure, where it however must be made sure that the electrode poles 102 does not penetrate through the septum M and does not reach into the left ventricle LV. In addition, also the second electrode pole 103 shall establish a desired coupling to tissue.

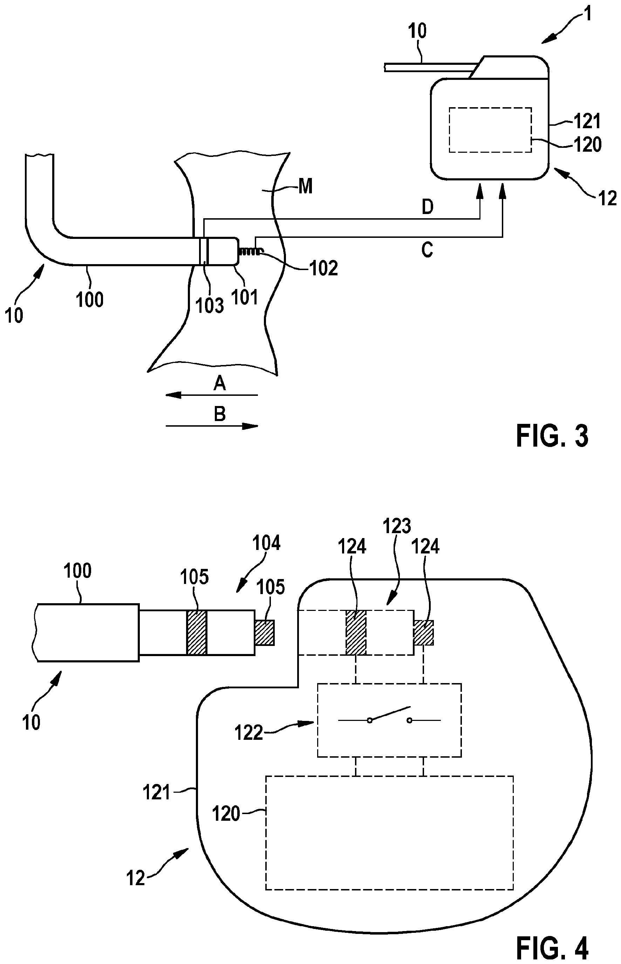

Referring now to Fig. 3, in the shown embodiment the implantable medical stimulation device 1 comprises an electrode pole arrangement made up of altogether three electrode poles, a first electrode pole 102 being formed by the helical electrode pole on the distal end 101 of the lead body 100 of the electrode lead 10, a second electrode pole 103 being formed by the ring electrode on the lead body 100, and a third electrode pole 121 being formed by the housing of the generator device 12.

In the shown embodiment, the electrode lead 10 is configured as a bipolar lead comprising two electrode poles 102, 103. As shown in Fig. 4, the electrode lead 10 comprises a bipolar connector 104 having two electrical contact elements 105. The generator device 12 comprises a complementary bipolar connector 123 having two electrical contact elements 124. An electrical connection in between the electrode lead 10 and the generator device 12 is established by plugging the connectors 104, 123 into each other, such that the electrode poles 102, 103 of the electrode lead 10 are in operative connection with the processing circuitry 120 of the generator device 12.

As shown in Fig. 4, the generator device 12, in addition, comprises a switching device 122 which allows to selectively switch between different combinations of electrode poles of the electrode pole arrangement formed by the electrode poles 102, 103, 121 of the electrode lead 10 and the housing of the generator device 12. Hence, operation may be controlled to use different combinations of electrode poles 102, 103, 121 to output stimulation signals in order to perform a pacing action within a patient.

Namely, a first combination of electrode poles is formed by the first electrode pole 102 on the distal end 101 of the electrode lead 10 and the second electrode pole 103 arranged proximally with respect to the first electrode pole 102 on the lead body 100 of the electrode lead 10, the first electrode pole 102 herein serving as cathode and the second electrode pole 103 serving as anode. In this combination, the electrode poles 102, 103 span an excitation vector A, as illustrated in Fig. 3.

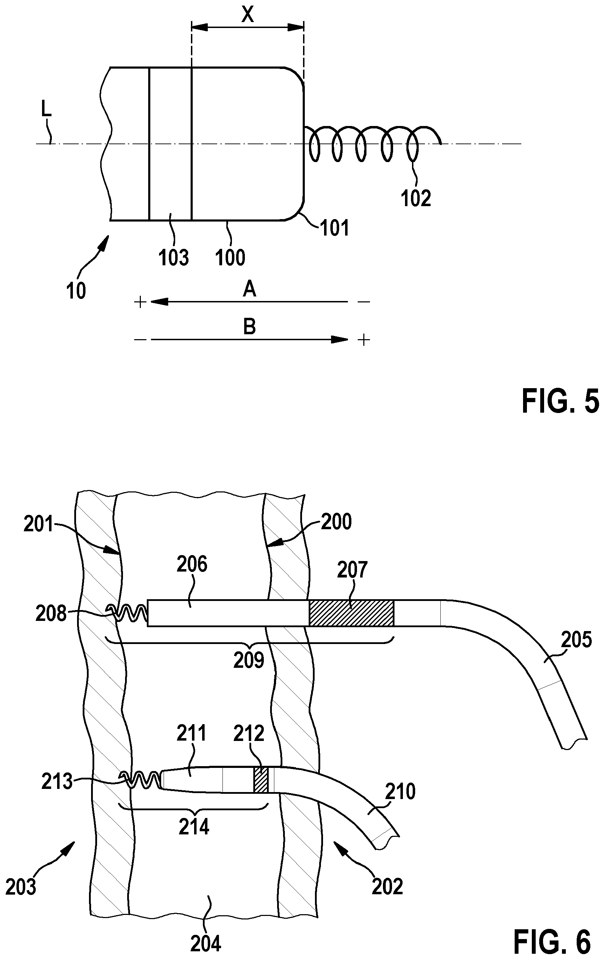

In addition, a second combination of electrode poles is formed by the first electrode pole 102 and the second electrode pole 103, wherein now the second electrode pole 103 serves as cathode and the first electrode pole 102 serves as anode, the electrode poles 102, 103 in this combination hence spanning an excitation vector B as illustrated in Fig. 3. The excitation vector B, as illustrated in Fig. 3 and in addition in Fig. 5, has an inverse polarity with respect to the excitation vector A.

Further, a third combination of electrode poles is formed by the first electrode pole 102, serving as cathode, and the electrode pole 121 formed by the housing of the generator device 12, the electrode poles 102, 121 spanning an excitation vector C as illustrated in Fig. 3.

A fourth combination of electrodes poles is formed by the second electrode pole 103, serving as cathode, on the lead body 100 and the third electrode pole 121, serving as anode, formed by the housing of the generator device 12, this combination of electrode poles spanning an excitation vector D as illustrated in Fig. 3.

Hence, in the example of Figs. 3 and 4, the electrode poles 102, 103, 121 of the electrode pole arrangement may be combined to form different combinations of electrode poles, wherein the processing circuitry 120 may selectively, using the switching device 122 as illustrated in Fig. 4, switch between the different combinations of electrode poles in order to output a generated stimulation signal.

For example, the first electrode port 102 may be configured to rest, in an implanted state, in a depth within the septum M of the patient's heart such that the first electrode pole 102 reaches towards the left bundle branch LBB and effectively is coupled to the left bundle branch LBB. The second electrode pole 103, in turn, may be buried within tissue in the right portion of the septum M, such that the electrode pole 103 rests in the vicinity to the right bundle branch RBB and may effectively be coupled to the right bundle branch RBB. When using the first or third combination of electrode poles, in which in each case the first electrode pole 102 serves as cathode, an effective stimulation in particular at the left bundle branch LBB may be achieved. In turn, when using the second or fourth combination of electrode poles, in which in each case the second electrode pole 103 is used as cathode, an effective stimulation in particular at the right bundle branch RBB may be achieved. By employing different combinations of electrode poles and hence by effectively using different excitation vectors A-D, a differentiated spatial excitation of portions of the conductive structure of the patient's heart may be established, such that for example a biventricular stimulation both in the right ventricle and the left ventricle may be achieved.

The processing circuitry 120 of the generator device 12, in one embodiment, is configured to generate stimulation signals during a cardiac cycle which serve to both provide for a right ventricular stimulation and a left ventricular stimulation using a single bipolar lead 10. For example, a first stimulation signal may be generated to be output by the first combination of electrode poles, and at a defined time distance a second stimulation signal within the same cardiac cycle may be generated to be output by the second combination of electrode poles, such that by means of the different combinations a stimulation in the left ventricle and the right ventricle is achieved. The order of stimulation signals herein may be selectively chosen in order to establish a synchronized cardiac activity.

A time distance between different stimulation signals to be output in a cardiac cycle using different combinations of electrode poles may be adaptively set by the processing circuitry 120 during operation.

In one embodiment, the processing circuitry 12 is configured to switch from a currently used combination of electrode poles to another combination of electrode poles if a capture loss or a change in a cardiac sense signal is identified. If for example a capture loss is detected, the processing circuitry 120, by means of the switching device 122, may switch from a currently used combination of electrode poles to another combination of electrode poles. If for example a broadening of a QRS complex based on sensed electrocardiogram signal is identified, likewise the processing circuitry 120 may switch from a currently used combination of electrode poles to another combination of electrode poles for outputting a stimulation signal.

A monitoring of a potential capture loss and/or a monitoring of a QRS complex may be cyclically conducted by the processing circuitry 120 in order to adaptively switch from one combination of electrode poles to another based on an identification of a potential capture loss or a change in cardiac sense signal.

Referring now to Fig. 5, the first electrode pole 102 and the second electrode pole 103 are arranged at an axial distance X with respect to one another, measured along a longitudinal axis L along which the lead body 100 of the electrode lead 10 generally extends. The first electrode pole 102, formed by a helical screw, longitudinally protrudes from the distal end 101 of the lead body 100. The second electrode pole 103 may for example be formed by a ring electrode circumferentially extending about the lead body 100. The axial distance X may for example be smaller than 10 mm, for example in a range between 7 mm and 8 mm.

In different combinations of electrode poles the first electrode pole 102 may serve as cathode and hence may have a negative polarity, whereas in other combinations the second electrode pole 103 may serve as cathode and hence may have a negative polarity, as illustrated in Fig. 5 according to the excitation vectors A, B.

Figure 6 illustrates a simplified cross-sectional view of a septum 204 of a heart, which is the anatomical wall between the right ventricle (RV) 202 and left ventricle (LV) 203. The right bundle branch (RBB) 200 and the left bundle branch (LBB) 201 are embedded in the septum 204 and are depicted as left and right vertical borders of the septum. Stimulation lead 205 with cylindrical shape tip portion 206, known from the prior art, has been screwed into septum 204 from the RV 202 in direction of the LV 203 using helical tip electrode 208. Ring electrode 207 is located further proximal the lead body. As it can be seen in Fig. 6, tip electrode 208 may stimulate the LBB 201, whereby ring electrode 207 is located in the RV 202 without tissue contact to the RBB 200. In other words, pole to pole distance 209 of electrode lead 205 is too great to allow stimulation of the RBB 200 via ring electrode 207, since the septum of most of the patients is not sufficiently thick. Typically known pole to pole distances for stimulation leads as 205 with cylindrical shaped tip range from about 12 mm and more. It is actually possible to use stimulation lead 205 for stimulating the RBB 200 using ring electrode 207 either in patients having a thicker septum, or by introducing the stimulation electrode 207 at an angle at during implantation. However, commonly known ring electrodes as ring electrode 209 have a comparatively large area, so that stimulation is energy consuming, since large currents need to be applied to achieve reach stimulation threshold due to lower current densities.

Further referring to Fig. 6, and according to embodiments of the present invention, a stimulation lead 210 having a conical shape tip portion 211 and reduced pole to pole distance 214 is shown. Stimulation lead 210 having a conical shape tip portion 211 has been screwed into septum 204 from the RV 202 in direction of the LV 203 using helical tip electrode 213. Ring electrode 212 is located further proximal the lead body. As it can be seen in Fig. 6, tip electrode 213 may stimulate the LBB 201, whereby ring electrode 214 is located in the septum 204, therefor able to achieve electrical stimulation of the RBB 200. In other words, pole to pole distance 214 of electrode lead 210 is dimensioned in a way to allow stimulation of the RBB 200 via ring electrode 212, since the pole to pole distance 214 is adapted according to typical. The pole-to-pole distance for stimulation lead 210 according to the invention range from about 12 mm or less, in particular 10 mm or less, in particular 9,7mm. Moreover, the conical shape tip portion 214 facilitates the implantation procedure, since the conical shape enables penetration and forwarding of the electrode tip into tissue using less force compared to a cylindric shape tip 206, since more pressure is applied at the tip due to a smaller tip surface. Additionally, ring electrode 212 has a reduced surface compared to ring electrode 207, allowing higher current densities and therefore more energy efficient pacing.

The idea underlying the invention is not limited to the embodiments described above, but may be implemented in an entirely different fashion.

An electrode pole arrangement of an implantable medical stimulation device may comprise two or more electrode poles, e.g. three electrode poles or more than three electrode poles, wherein it may be selectively switched between different combination of the available electrode poles.

An implantable medical stimulation device may be configured for providing for a left bundle branch area pacing, but may, alternatively or in addition, implement different pacing functions.

### List of Reference Numerals

- 1: Implantable medical stimulation device
- 10: Lead
- 100: Lead body
- 101: Distal end
- 102: Electrode pole
- 103: Electrode pole
- 104: Connector
- 105: Contact elements
- 11: Lead
- 12: Generator
- 120: Processing circuitry
- 121: Housing
- 122: Switching device
- 123: Connector
- 124: Contact elements
- 200: Right bundle branch (RBB)
- 201: Left bundle branch (LBB)
- 202: Right ventricle (RV)
- 203: Left ventricle (LV)
- 204: Septum
- 205: Stimulation lead with cylindrically shaped tip
- 206: Cylindrical shape tip portion
- 207: Ring electrode
- 208: Helical tip electrode
- 209: Pole to pole distance
- 210: Simulation lead with conically shaped tip211 Conical shape tip portion
- 212: Ring electrode
- 213: Helical tip electrode
- 214: Pole to pole distance
- A, B, C, D: Excitation vectors
- AVN: Atrioventricular node
- H: Heart
- HIS: HIS bundle branch
- L: Longitudinal axis
- LA: Left atrium
- LBB: Left bundle branch
- LV: Left ventricle
- M: Intra-cardiac tissue (myocardium)
- RA: Right atrium
- RBB: Right bundle branch
- RV: Right ventricle
- V: Superior vena
- X: Distance

## Claims

1. An implantable medical stimulation device (1) for performing a cardiac pacing, comprising:
a generator device (12) comprising processing circuitry (120) for processing electrical signals,
an electrode lead (10, 11) connected to the generator device (12) and extending from the generator device (12), the electrode lead (10) comprising a lead body (100) forming a distal end (101) to be arranged, during implantation of the electrode lead (10), on cardiac tissue within a patient's heart (H), and
an electrode pole arrangement comprising at least a first electrode pole (102) and a second electrode pole (103), wherein the first electrode pole (102) is configured to penetrate cardiac tissue during implantation, wherein the second electrode pole (103) is arranged on the lead body (100) at a location proximal to the first electrode pole (102),
**characterized in that** the processing circuitry (120) is configured to generate a stimulation signal and to selectively provide said stimulation signal to one out of at least two combinations of electrode poles of the electrode pole arrangement, wherein in a first combination of the at least two combinations of electrode poles the first electrode pole (102) is used as cathode and in a second combination of the at least two combinations of electrode poles the second electrode pole (103) is used as cathode.

2. The implantable medical stimulation device (1) according to claim 1, wherein the first electrode pole (102) comprises a helical shape arranged on the distal end (101) of the lead body (100) configured to be screwed into cardiac tissue during implantation, and/or wherein the first electrode pole (102) comprises a needle shape arranged on the distal end (101) of the lead body (100).

3. The implantable medical stimulation device (1) according to claim 1, **characterized in that** in said first combination the second electrode pole (103) is used as anode, and wherein in said second combination the first electrode pole (102) is used as anode.

4. The implantable medical stimulation device (1) according to one of claims 1 to 3, **characterized in that** said electrode pole arrangement comprises a third electrode pole (121) formed by a housing of the generator device (12).

5. The implantable medical stimulation device (1) according to claim 4, **characterized in that** in at least one out of the at least two combinations of electrode poles of the electrode pole arrangement the third electrode pole (121) is used as anode.

6. The implantable medical stimulation device (1) according to claim 4 or 5, **characterized in that** in a third combination of the at least two combinations of electrode poles the first electrode pole (102) is used as cathode and the third electrode pole (121) is used as anode, and/or in a fourth combination of the at least two combinations of electrode poles the second electrode pole (103) is used as cathode and the third electrode pole (121) is used as anode.

7. The implantable medical stimulation device (1) according to one of the preceding claims, **characterized in that** the first electrode pole (102) is configured to rest, in an implanted state of the implantable medical stimulation device (1), within cardiac tissue of the septum (M) of the patient's heart in the vicinity of the left bundle branch (LBB), and the second electrode pole (103) is configured to rest, in an implanted state of the implantable medical stimulation device (1), within cardiac tissue of the septum (M) in the vicinity of the right bundle branch (RBB).

8. The implantable medical stimulation device (1) according to one of the preceding claims, **characterized in that** the generator device (12) comprises a switching device (122) to selectively switch between the different combinations of the at least two combinations of electrode poles of the electrode pole arrangement.

9. The implantable medical stimulation device (1) according to one of the preceding claims, **characterized in that** the processing device (120) is configured to sequentially, within a cardiac cycle, generate a first stimulation signal to be output using said first combination and a second stimulation signal to be output using said second combination.

10. The implantable medical stimulation device (1) according to claim 9, **characterized in that** the processing device (120) is configured to generate said first stimulation signal and said second stimulation signal at a defined time distance.

11. The implantable medical stimulation device (1) according to claim 9 or 10, **characterized in that** said first stimulation signal is configured to provide for a stimulation in one of the ventricles of the patient's heart and said second stimulation signal is configured to provide for a stimulation in the other of the ventricles of the patient's heart.

12. The implantable medical stimulation device (1) according to one of the preceding claims, **characterized in that** the processing device (120) is configured to switch from one out of the at least two combinations of electrode poles of the electrode pole arrangement to another out of the at least two combinations of electrode poles of the electrode pole arrangement based on a processing of at least one sense signal sensed using the electrode pole arrangement.

13. The implantable medical stimulation device (1) according to one of the preceding claims, **characterized in that** the first electrode pole (102) and the second electrode pole (103) are arranged with respect to each other at a distance (X), measured along a longitudinal axis along which the lead body (100) generally extends, less than 10 mm, preferably in a range between 7 to 8 mm.

14. The implantable medical stimulation device (1) according to one of the preceding claims, **characterized in that** the generator device (12) comprises a first bipolar connector (123) having two first electrical contact elements (124) and the electrode lead (10) comprises a second bipolar connector (104) comprising two second electrical contact elements (105), wherein the first connector (123) and the second connector (104) are connected for establishing an operative connection in between the processing circuitry (120), the first electrode pole (102) and the second electrode pole (103).

15. A method for operating an implantable medical stimulation device (1) for performing a cardiac pacing, the method comprising:
providing a generator device (12) comprising processing circuitry (120) for processing electrical signals,
providing an electrode lead (10, 11) connected to the generator device (12) and extending from the generator device (12), the electrode lead (10) comprising a lead body (100) forming a distal end (101) to be arranged, during implantation of the electrode lead (10), on cardiac tissue within a patient's heart (H), and
providing an electrode pole arrangement comprising at least a first electrode pole (102) and a second electrode pole (103), wherein the first electrode pole (102) is configured to penetrate cardiac tissue during implantation, wherein the second electrode pole (103) is arranged on the lead body (100) at a location proximal to the first electrode pole (102),
**characterized by** generating, using the processing circuitry (120), a stimulation signal and selectively providing said stimulation signal to one out of at least two combinations of electrode poles of the electrode pole arrangement, wherein in a first combination of the at least two combinations of electrode poles the first electrode pole (102) is used as cathode and in a second combination of the at least two combinations of electrode poles the second electrode pole (103) is used as cathode.
